# EUROPÄISCHE PATENTSCHRIFT

(11) **EP 3 949 781 B1**
(45) Veröffentlichungstag und Bekanntmachung des Hinweises auf die Patenterteilung: **03.04.2024**
(21) Anmeldenummer: 21188233.7
(22) Anmeldetag: 28.07.2021
(51) Int. Cl.: A42B 3/14

(54) **KOPFAUFHÄNGUNG FÜR EINE KOPFBEDECKUNG, ATEMSCHUTZHAUBE MIT EINER KOPFBEDECKUNG UND VERFAHREN ZUM BEFESTIGEN EINER KOPFBEDECKUNG**
HEAD SUSPENSION FOR HEADGEAR, RESPIRATOR HOOD WITH HEADGEAR AND METHOD FOR FIXING HEADGEAR
ACCROCHAGE POUR UN COUVRE-TÊTE, CAPUCHON DE PROTECTION RESPIRATOIRE DOTÉ D'UN COUVRE-TÊTE ET PROCÉDÉ DE FIXATION D'UN COUVRE-TÊTE

(30) Priorität: 07.08.2020 DE 102020120950
(43) Veröffentlichungstag der Anmeldung: 09.02.2022
(73) Patentinhaber: SATA GmbH & Co. KG, 70806 Kornwestheim (DE)
(72) Erfinder: Brose, Jens, 74354 Ottmarsheim (DE); Schönemann, Marco, 73240 Wendlingen (DE)

(56) Entgegenhaltungen:
- CN-Y- 2 820 148
- DE-A1- 3 543 341
- US-A1- 2006 156 448
- US-A1- 2012 144 565
- US-A1- 2015 250 251

## Beschreibung

Die Erfindung betrifft eine Kopfaufhängung für eine Kopfbedeckung, vorzugsweise für eine Atemschutzhaube, umfassend ein Kopfband mit einem Vorderkopfband, welches die Vorderseite des Kopfes eines Trägers umläuft und ein Hinterkopfband, welches sich auf der Hinterseite des Kopfes des Trägers befindet, wobei das Hinterkopfband über zwei Drehgelenke mit dem Vorderkopfband verbunden ist.

Des Weiteren betrifft die Erfindung eine Atemschutzhaube mit einer derartigen Kopfaufhängung, sowie ein Verfahren zum Befestigen einer Kopfbedeckung mit einer solchen Kopfaufhängung.

Solche Kopfaufhängungen dienen der Befestigung einer Kopfbedeckung wie beispielsweise einer Atemschutzhaube, eines Schweißerhelms oder eines Schutzhelms am Kopf eines Trägers und bilden so das Verbindungsglied zwischen dem Kopf des Nutzers und der Kopfbedeckung. Meist besteht eine solche Kopfaufhängung aus mehreren Teilen, wobei es für die Anpassung an den Kopf des Trägers von Vorteil ist, wenn der Winkel eines hinteren Teils der Kopfaufhängung relativ zu einem vorderen Teil des Kopfbandes, beziehungsweise dem Kopf des Trägers, verstellbar ist. Um die Kopfaufhängung und somit die Kopfbedeckung am Kopf des Trägers zu fixieren, ist meist ein Längenverstellmittel vorgesehen mit dem der Innenumfang der Kopfaufhängung verstellt werden kann. Durch eine Reduktion des Innenumfangs wird ein Kraftschluss zwischen dem Kopf des Trägers und der Kopfaufhängung erzielt.

Eine Kopfaufhängung, gemäß eingangs erwähnter Bauart, ist z. B. in der JP5899836B2 offenbart. Die dort beschriebene Kopfaufhängung weist ein Vorderkopfband und ein Hinterkopfband auf. Diese sind beidseitig über Drehgelenke miteinander verbunden. Am Hinterkopfband ist eine Verzahnung und ein gegenüberliegendes Gegenelement vorgesehen. Das Vorderkopfband weist einen Stift auf, der sich im Raum zwischen Verzahnung und dem Gegenelement befindet. Der Raum zwischen Verzahnung und dem Gegenelement ist im Bereich der Spitzen der Verzahnung kleiner als der Durchmesser des Stiftes. Diese Bereiche bilden Engstellen, an denen beim Verdrehen der Drehgelenke eine erhöhte Kraft aufgewendet werden muss, damit der Stift die Engstellen überwindet. Durch diesen Aufbau wurde eine Struktur geschaffen, die dazu dient, den Winkel des Hinterkopfbandes relativ zum Vorderkopfband in einer eingestellten Position zu halter

US 2015/250251 offenbart eine Kopfaufhängung gemäß dem Oberbegriff des Anspruchs 1.

Der Erfindung liegt die Aufgabe zugrunde, eine Kopfaufhängung mit verstellbarem Winkel zwischen Vorderkopfband und Hinterkopfband bereitzustellen, wobei der Winkel mit hoher Funktionssicherheit festgestellt ist.

Die Aufgabe wird durch eine Kopfaufhängung mit den Merkmalen von Anspruch 1 gelöst.

Die erfindungsgemäße Kopfaufhängung für eine Kopfbedeckung, vorzugsweise für eine Atemschutzhaube, umfasst ein Kopfband mit einem Vorderkopfband, welches die Vorderseite des Kopfes eines Trägers umläuft und ein Hinterkopfband, welches sich auf der Hinterseite des Kopfes des Trägers befindet. Das Hinterkopfband ist über zwei Drehgelenke mit dem Vorderkopfband verbunden. Die Drehgelenke sind in einer jeweiligen Verstellstellung zumindest bereichsweise frei drehbar und in einer jeweiligen Arretierstellung nicht drehbar.

Da die Drehgelenke in der Arretierstellung, im Unterschied zum Stand der Technik, nicht drehbar sind, ist sichergestellt, dass sich die Winkelstellung nicht unerwünscht verstellt. Dies ist sowohl im getragenen Zustand wie auch im nicht getragenen Zustand der Fall. Im getragenen Zustand ist durch einen unverstellbaren Winkel zwischen Hinterkopfband und Vorderkopfband eine besonders vorteilhafte Kraftverteilung zwischen Kopfband und Kopf des Trägers gewährleistet. Hierdurch kann die Befestigung am Trägerkopf und der Tragekomfort erhöht werden. In ungetragenem Zustand bleibt der Winkel, den der Träger passend eingestellt hat, erhalten wodurch ein erneutes Einstellen unnötig wird.

Für die Befestigung der Kopfaufhängung auf dem Kopf des Trägers ist es von Vorteil, wenn die Kraft auf der Hinterseite des Trägerkopfes im unteren Bereich des Hinterkopfes wirkt, da die Kopfaufhängung dadurch zusätzlich nach unten gezogen wird. Dies ist nur möglich, wenn das Hinterkopfband in einem Winkel vom Vorderkopfband relativ zum Kopf des Trägers nach unten steht und dieser Winkel sich nicht verändert, wenn das Kopfband am Kopf des Trägers befestigt beziehungsweise festgezogen wird. Wäre der Winkel nicht arretiert, würde sich das Hinterkopfband bei Krafteinwirkung in Richtung der Transversalebene bewegen, die mittig durch das Vorderkopfband verläuft, was die beschriebene vorteilhafte Kraftverteilung behindern oder verhindern würde. Somit führt das Arretieren des Winkels zwischen Vorderkopfband und Hinterkopfband dazu, dass sich das Hinterkopfband bei Krafteinwirkung nicht nach oben bewegt.

Durch das Arretieren des Winkels zwischen Vorderkopfband und Hinterkopfband ist es außerdem möglich, das Kopfband an verschiedenste Kopfformen beziehungsweise Kopfgrößen anzupassen und dennoch einen komfortablen Sitz des Kopfbandes über längere Zeit zu gewährleisten.

Beide Drehgelenke können unabhängig voneinander von der Verstellstellung in die Arretierstellung überführt werden.

Bezüglich der Verbesserung des Tragekomforts ist es besonders vorteilhaft, wenn das Vorderkopfband aus einer weicheren Komponente als das Hinterkopfband gefertigt ist. Insbesondere ist das Vorderkopfband aus einem flexiblen, aber formstabilen Material gefertigt, das sich besonders gut an den Kopf anschmiegt. Das Hinterkopfband hingegen ist aus einem weniger elastischem Material gefertigt, das verschleißfester ist. Dies ist insbesondere von Vorteil, wenn das Hinterkopfband ein Längenverstellmittel aufweist, das in diesem Fall weniger verschleißanfällig ist, wobei dennoch ein optimaler Tragekomfort gewährleistet wird. Insbesondere ist hierzu im Oberkopfbereich ein noch weicheres, vorzugsweise gummielastisches, Material vorgesehen, das ein Oberkopfband bildet. Durch dieses Oberkopfband wird die Kopfbedeckung senkrecht zum Kopf des Trägers stabilisiert beziehungsweise bildet ein Widerlager gegen die nach unten wirkenden Kräften, die durch das Gewicht der Kopfbedeckung entstehen. Somit besteht die Kopfaufhängung aus drei verschiedenen Materialien mit unterschiedlicher Härte und/oder Elastizität. Besonders vorteilhaft ist hierbei, wenn das Material des Vorderkopfbandes eine Shorehärte von weniger als 80 Shore-D, insbesondere weniger als 75 Shore-D aufweist, besonders bevorzugt weniger als 60 Shore-D.

Eine solche vorteilhafte Befestigung kann beispielsweise dadurch erzielt werden, dass die Drehgelenke jeweils durch eine Relativbewegung, insbesondere durch eine geradlinige Relativbewegung, der Drehachse des jeweiligen Drehgelenks, vorzugsweise über ein Gleiten in einem Langloch und/oder einer Längsnut, von der Verstellstellung in die Arretierstellung und zurück überführbar sind. Bei einer solchen Relativbewegung bewegt sich ein Hinterkopfband-Gelenkteil relativ zu einem Vorderkopfband-Gelenkteil. Beim Hinterkopfband-Gelenkteil handelt es sich um die Teile der Drehgelenke am Hinterkopfband und beim Vorderkopfband-Gelenkteil um die Teile der Drehgelenke am Vorderkopfband. Vorzugsweise verändert sich bei der Relativbewegung die Position der Drehachsen relativ zum Vorderkopfband nicht, wohingegen sich die Position der Drehachsen relativ zum Hinterkopfband verschiebt. Alternativ zu einer gradlinigen Relativbewegung kann es sich auch um eine translatorische Bewegung handeln, die nicht geradlinig ist. Durch das beschriebene Verschieben der Drehachsen können die Drehgelenke zum größten Teil einstückig mit dem Vorderkopfband und dem Hinterkopfband gefertigt werden, wodurch zusätzliche Teile eingespart werden.

Außer den Teilen der beiden Drehgelenke, die einstückig mit dem Vorderkopfband oder dem Hinterkopfband gefertigt sind, wird lediglich ein durchgreifendes Verbindungselement benötigt, wobei sich das durchgreifende Verbindungselement durch das Drehgelenk hindurch erstreckt und das Drehgelenk durch beidseitige Widerlager am Verbindungselement zusammenhält. Das durchgreifende Verbindungselement besteht vorzugsweise aus zwei einzelnen Elementen.

Die Drehgelenke sind jeweils durch eine Relativbewegung eines Teils des jeweiligen Drehgelenks am Hinterkopfband in Richtung der Blickrichtung des Trägers von der Arretierstellung in die Verstellstellung überführbar. Wenn das Kopfband in Gegenrichtung zurückbewegt wird, findet eine Rückführung des Drehgelenks in Arretierstellung statt, in welcher sich der Winkel des Hinterkopfbandes relativ zum Vorderkopfband nicht mehr verändern lässt. Unter Blickrichtung ist die Richtung zu verstehen, in die der Träger bei verwendungsgemäßem Gebrauch der Kopfaufhängung beziehungsweise der Kopfbedeckung blickt. Die Blickrichtung weist somit vom Hinterkopfband aus zum Vorderkopfband, und verläuft vorzugsweise in etwa parallel zum Vorderkopfband. Durch eine Arretierung der jeweiligen Drehgelenke entgegen der Blickrichtung wird gewährleistet, dass sich das Drehgelenk nicht selbsttätig in die Verstellstellung bewegt, wenn die Kopfbedeckung getragen wird.

Vorzugsweise weist das Hinterkopfband ein Längenverstellmittel auf, über welches die Länge und damit der Innenumfang des Kopfbandes verstellt werden kann. Durch ein Verkürzen des Innenumfangs kann das Kopfband am Kopf des Trägers befestigt werden, wobei eine Kraft vom Kopfband auf den Kopf des Trägers einwirkt, die zu einem Kraftschluss zwischen Kopfband und Kopf führt. Ein solches Längenverstellmittel bietet außerdem den Vorteil, dass der Innenumfang des Kopfbandes an den Umfang des Trägerkopfes angepasst beziehungsweise eingestellt werden kann.

Des Weiteren können die Drehgelenke jeweils ein Sicherungsmittel, insbesondere in Form einer Engstelle in einer Längsnut oder einem Langloch, aufweisen, das der Überführung des jeweiligen Drehgelenks von der Verstellstellung in die Arretierstellung mit einer Sicherungskraft entgegenwirkt, die aufgebracht werden muss, um das Sicherungsmittel zu überwinden und das Drehgelenk von der Arretierstellung in die Verstellstellung zu überführen. Durch ein solches Sicherungsmittel wird gewährleistet, dass die Drehgelenke automatisch in der Arretierstellung beziehungsweise der Verstellstellung gehalten werden, auch wenn die Kopfaufhängung nicht getragen wird.

Besonders bevorzugt ist ein Ausführungsbeispiel, bei welchem die Drehgelenke jeweils eine Winkelstelleinrichtung aufweisen, über welche diskrete Winkelstellungen des Hinterkopfbandes relativ zum Vorderkopfband, vorzugsweise zwei oder drei diskrete Winkelstellungen, fest definiert sind. Vorzugsweise kommen die Winkelstelleinrichtungen vor den Arretiermitteln in Eingriff, wenn die Drehgelenke von der Verstellstellung in die Arretierstellung überführt werden. Durch diese Ausgestaltung wird ein einfaches Mittel bereitgestellt, um den Winkel zwischen Hinterkopfband und Vorderkopfband reproduzierbar einzustellen. Besonders vorteilhaft ist es, wenn sich der Winkel zwischen Vorderkopfband und Hinterkopfband in einem Bereich von 30° verstellen lässt, wobei drei Winkelstellungen vorgesehen sind.

Die Winkelstelleinrichtungen können durch längliche Zapfen gebildet sein, die vom Vorderkopfband und Hinterkopfband abstehen, vorzugsweise wobei die Vorderkopfbandzapfen in der Verstellstellung versetzt zu den Hinterkopfbandzapfen angeordnet sind, und wobei Vorderkopfbandzapfen und Hinterkopfbandzapfen in der Arretierstellung ineinandergreifen. Sowohl Vorderkopfbandzapfen wie auch Hinterkopfbandzapfen können einteilig mit dem Kopfband gefertigt werden, was zu weniger Einzelteilen führt und die Montage erleichtert.

Besonders vorteilhaft ist, wenn Arretiermittel vorgesehen sind, die durch eine Verzahnung gebildet werden, vorzugsweise wobei die Verzahnung den Winkelstelleinrichtungen bezüglich der Drehachse des Drehgelenks gegenüberliegt. Eine Verzahnung hat den Vorteil, dass Drehkräfte besonders gut aufgenommen werden können.

Im Falle eines besonders bevorzugten Ausführungsbeispiels, sind die Drehgelenke im Wesentlichen mittig zwischen Oberkante und Unterkante des Vorderkopfbands angeordnet. Durch diese Gestaltung kann eine optimale Passform für unterschiedliche Kopfgrößen und Kopfformen erreicht werden.

Vorteilhaft ist, dass die Drehgelenke jeweils einen Drehkörper, der vorzugsweise gegenüber dem Vorderkopfband vorsteht, und zwei sich überschneidende Aussparungen, vorzugsweise am Hinterkopfband, aufweisen. Ein solcher Drehkörper beziehungsweise solche Aussparungen können einstückig mit dem Vorderkopfband oder dem Hinterkopfband gefertigt sein, wodurch weniger Einzelteile benötigt werden.

In dieser vorteilhaften Ausführung liegt der, im wesentlichen zylinderförmige, Drehkörper in der Arretierstellung innerhalb einer ersten Aussparung der sich überschneidenden Aussparungen und in der Verstellstellung innerhalb einer zweiten Aussparung der sich überschneidenden Aussparungen. In der Verstellstellung dient die zweite Aussparung als Drehführung für den Drehkörper. Der Drehkörper weist in einer besonders bevorzugten Ausgestaltung zwei Ausnehmungen auf, die in Form eines Langloches entlang einer gebogenen Linie verlaufen, die innerhalb des Drehkörpers entlang des Außenumfangs verläuft. Diese Ausnehmungen dienen dazu, einen elastischen Bereich zwischen den Ausnehmungen und dem Außenumfang des Drehkörpers zu schaffen, der nachgibt, wenn sich das Sicherungsmittel über diesen Bereich bewegt, wenn die Drehgelenke von der Arretierstellung in die Verstellstellung überführt werden.

Im Übrigen können die Drehgelenke jeweils einen Drehkörper aufweisen, der an der dem Hinterkopfband gegenüberliegenden Seite, eine Außenverzahnung aufweist. Zudem sind Aussparungen auf der dem Hinterkopfband zugewandten Seite mit einer Innenverzahnung versehen, die in Arretierstellung mit der Außenverzahnung in Eingriff steht und zusammen Arretiermittel bilden. Durch die Verzahnung kann die Kraft zwischen Vorderkopfband und Hinterkopfband besonders vorteilhaft übertragen werden.

Die Drehgelenke können jeweils einen Drehkörper aufweisen, der an der Seite, die dem Hinterkopfband zugewandt ist, eine Drehkörperaussparung aufweist. Diese Drehkörperaussparung kann einen Teil von Winkelstelleinrichtungen aufnehmen, die am Hinterkopfband-Gelenkteil vorstehen und beim Überführen der Drehgelenke von der Arretierstellung in die Verstellstellung in diese Drehkörperaussparung eingeschoben werden. Hierbei bildet zumindest eine Seite der Drehkörperaussparung einen Endanschlag für die Drehbewegung des jeweiligen Drehgelenks, wenn dieses in der Verstellstellung verdreht wird. Eine solche Drehkörperaussparung stellt auf besonders einfache und platzsparende Weise eine Begrenzung für die Verstellung des Winkels zwischen Vorderkopfband und Hinterkopfband dar.

Besonders bevorzugt ist die Verwendung der beschriebenen Kopfaufhängung für eine Atemschutzhaube. Eine Verwendung einer beschriebenen Kopfaufhängung in diesem Bereich bietet den Vorteil eines verbesserten Tragekomforts bei maximaler Anpassung an unterschiedliche Kopfformen und Kopfgrößen.

Ebenfalls als vorteilhaft wird das Verfahren zum Befestigen einer beschriebenen Kopfbedeckung angesehen. Hierbei stellt der Träger die Winkelstellung des Hinterkopfbandes relativ zum Vorderkopfband entsprechend seiner Kopfform und/oder Kopfgröße ein und überführt die Drehgelenke anschließend über eine Relativbewegung, insbesondere eine gradlinige Relativbewegung, der Drehgelenke, von der Verstellstellung in die Arretierstellung. Insbesondere dient das Verfahren der Befestigung einer Atemschutzhaube. Durch das beschriebene Verfahren wird die Einstellbarkeit des Winkels zwischen Vorderkopfband und Hinterkopfband besonders vereinfacht.

Besonders vorteilhaft ist es bei einem solchen Verfahren, wenn der Träger die Drehgelenke von der Verstellstellung in die Arretierstellung überführt, indem er die Länge und damit den Innenumfang des Kopfbandes über das Längeneinstellmittel reduziert. Somit kann das Einstellen des Hinterkopfbandes erfolgen, wenn die Kopfbedeckung auf dem Kopf des Trägers sitzt, wobei es dem Träger erleichtert wird, die optimale Stellung des Hinterkopfbandes zu finden.

Die Erfindung wird nachfolgend anhand beispielhafter Ausführungsbeispiele erläutert. Die Figuren zeigen:
**Fig. 1** eine perspektivische Ansicht einer Kopfaufhängung,
**Fig. 2** eine ausschnittsweise Ansicht eines Drehgelenks in Verstellstellung,
**Fig. 3** eine ausschnittsweise Schnittansicht eines Drehgelenks in Verstellstellung,
**Fig. 4** eine ausschnittsweise Ansicht eines Drehgelenks in Arretierstellung,
**Fig. 5** eine ausschnittsweise Schnittansicht eines Drehgelenks in Arretierstellung,
**Fig. 6** eine ausschnittsweise Ansicht eines Hinterkopfband-Gelenkteils,
**Fig. 7** eine Seitenansicht einer Kopfaufhängung auf dem Kopf eines Trägers,
**Fig. 8** eine perspektivische Ansicht einer Atemschutzhaube.

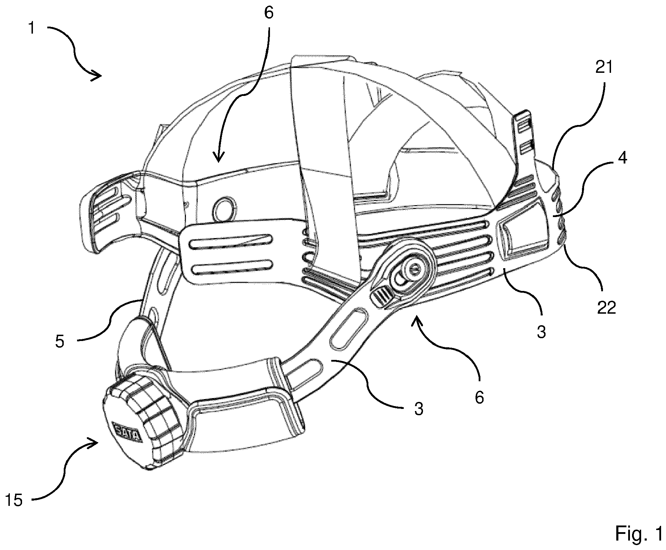

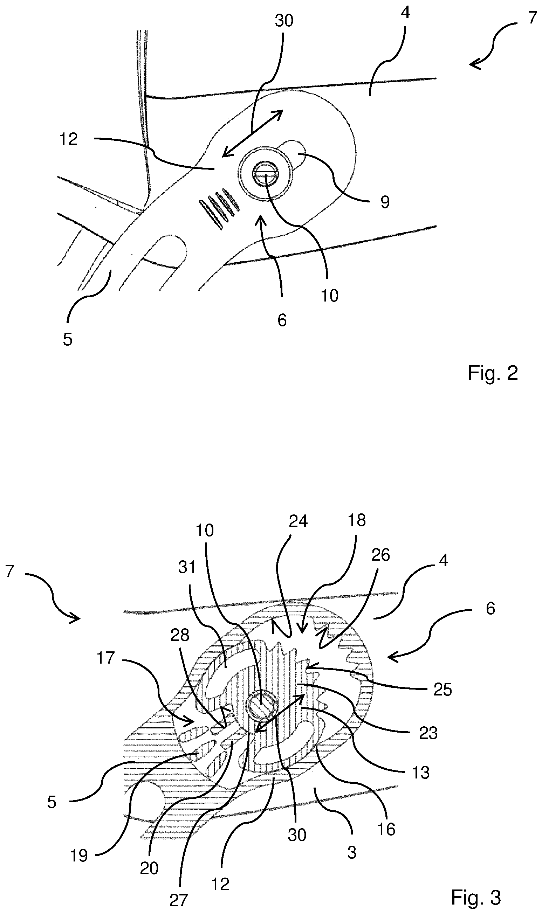

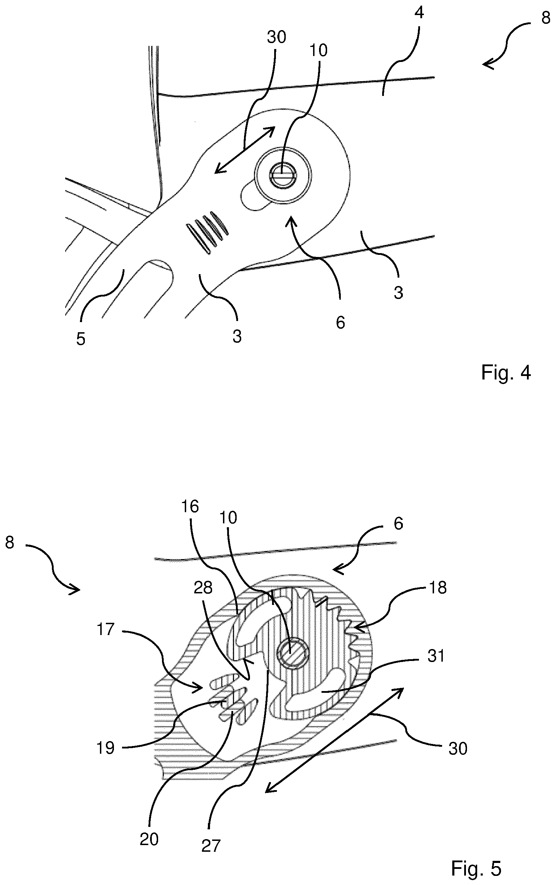

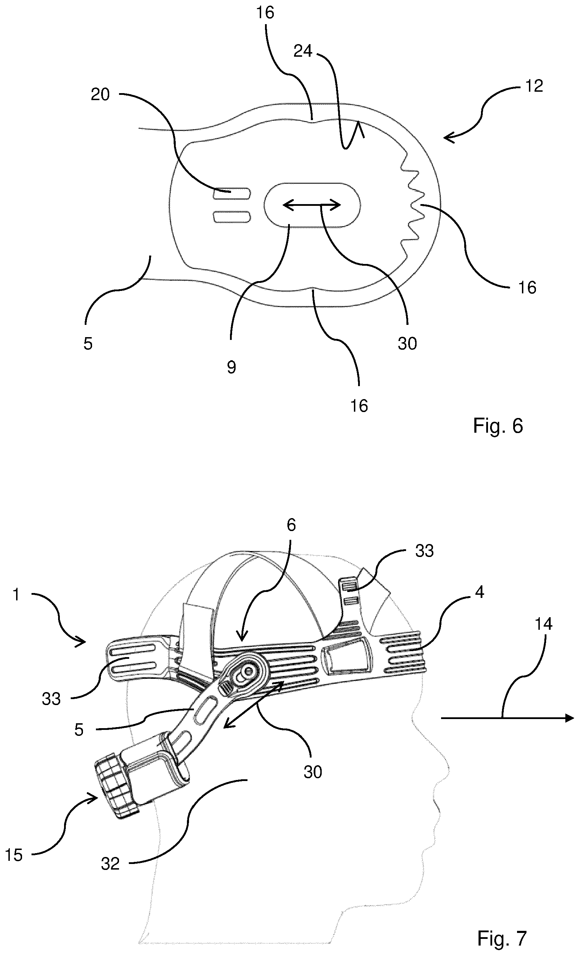

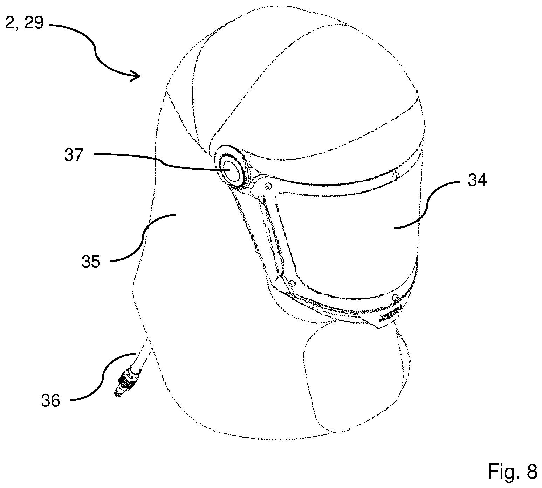

In den **Figuren 1** **und** **7** ist eine Kopfaufhängung 1 für eine Kopfbedeckung 2 aus Figur 8 gezeigt. Vorzugsweise handelt es sich bei der Kopfbedeckung 2 um eine Atemschutzhaube, wie sie ebenfalls in Figur 8 gezeigt ist. Die Kopfaufhängung 1 umfasst ein Kopfband 3 mit einem Vorderkopfband 4, welches die Vorderseite des Kopfes eines Trägers 32 umläuft, und einem Hinterkopfband 5, welches sich auf der Hinterseite des Kopfes des Trägers befindet, wobei das Hinterkopfband 5 über zwei Drehgelenke 6 mit dem Vorderkopfband 4 verbunden ist. Die Drehgelenke 6 sind in einer in Figur 2 und 3 gezeigten Verstellstellung 7 zumindest bereichsweise frei drehbar und in einer in Figur 4 und 5 gezeigten Arretierstellung 8 nicht drehbar.

Die Drehgelenke 6 der Kopfaufhängung 1 sind im Wesentlichen mittig zwischen Oberkante 21 und Unterkante 22 des Vorderkopfbands 4 angeordnet.

Das Hinterkopfband 5 weist ein Längenverstellmittel 15 auf, über welches die Länge und damit der Innenumfang des Kopfbandes 3 verstellt werden kann.

In den **Figuren 2 und 3** ist die Verstellstellung 7 gezeigt, in der die Drehgelenke 6 zumindest bereichsweise frei drehbar sind. Die **Figuren 4 und 5** zeigen die Drehgelenke 6 in der Arretierstellung 8.

Wie aus den **Figuren 2 bis 5** hervorgeht, sind die Drehgelenke 6 durch eine Relativbewegung 30, insbesondere durch eine geradlinige Relativbewegung 30, der Drehachse 10 des Drehgelenks 6, vorzugsweise über ein Gleiten in einem Langloch 9 und/oder einer Längsnut, von der Verstellstellung 7 in die Arretierstellung 8 und zurück überführt.

Bei einer solchen Relativbewegung 30 bewegt sich das Hinterkopfband-Gelenkteil 12 relativ zum Vorderkopfband-Gelenkteil 13. Die Relativbewegung erfolgt hierbei vorzugsweise in etwa entlang der Ebene, die senkrecht zur Drehachse 10 steht. Somit sind die Drehgelenke 6 jeweils durch eine Relativbewegung 30 eines Teils des jeweiligen Drehgelenks 6 am Hinterkopfband 5 in Richtung der, in Figur 7 gezeigten, Blickrichtung des Trägers 14 von der Arretierstellung 8 in die Verstellstellung 7 überführbar. Wenn eine Kopfbedeckung 2, insbesondere die Atemschutzhaube 29, mit einer Kopfaufhängung 1 am Kopf des Trägers 32 befestigt wird, erfolgt dies dementsprechend in der Art, dass der Träger die Winkelstellung des Hinterkopfbandes 5 relativ zum Vorderkopfband 4 entsprechend seiner Kopfform und/oder Kopfgröße einstellt und die Drehgelenke 6 anschließend über eine Relativbewegung 30, insbesondere eine gradlinige Relativbewegung 30 der Drehachse 10 von der Verstellstellung 7 in die Arretierstellung 8 überführt.

Der Träger kann die Drehgelenke 6 händisch von der Verstellstellung 7 in die Arretierstellung 8 überführen. Dies kann aber auch dadurch erfolgen, dass er die Länge und damit den Innenumfang des Kopfbandes 3 über das Längeneinstellmittel 15 reduziert. In diesem Fall werden die Drehgelenke 6 durch die Kraft, die sich durch ein Verkürzen des Kopfbandes 3 mittels der Längenverstellmittel 15 ergibt, selbsttätig in die Arretierstellung 8 gezogen.

Zum Feststellen der Drehgelenke 6 wird das Hinterkopfband 5, samt dem Hinterkopfband-Gelenkteil 12 in Richtung des Hinterkopfes des Trägers verschoben. Diese Arretierstellung ist in den **Figuren 4 und 5** gezeigt.

Die **Figuren 3** **und** **5** zeigen eine Schnittdarstellung der Drehgelenke 6 entlang der Ebene die senkrecht zur Drehachse 10 steht und mittig durch das Hinterkopfband-Gelenkteil 12 führt. Hier ist ersichtlich, dass die Drehgelenke 6 jeweils ein Sicherungsmittel 16, insbesondere in Form einer Engstelle in einer Längsnut oder einem Langloch, aufweisen, das der Überführung des jeweiligen Drehgelenks 6 von der Verstellstellung 7 in die Arretierstellung 8 mit einer Sicherungskraft entgegenwirkt, die aufgebracht werden muss, um das Sicherungsmittel 16 zu überwinden und das Drehgelenk 6 von der Arretierstellung 8 in die Verstellstellung 7 zu überführen.

Wie aus den **Figuren 3** **und** **5** weiterhin ersichtlich ist, weisen die Drehgelenke 6 in diesem Ausführungsbeispiel jeweils einen Drehkörper 23 auf, der vorzugsweise gegenüber dem Vorderkopfband 4 vorsteht. Zusätzlich sind zwei sich überschneidende Aussparungen 24 am Hinterkopfband 5 vorgesehen. Diese überschneidenden Aussparungen 24 sind in diesem Fall in etwa kreisrund und überschneiden sich derart, dass zwischen beiden kreisrunden Aussparungen 24 ein Bereich vorhanden ist, der schmaler als der Durchmesser der kreisrunden Aussparungen 24 ist. Dieser Bereich bildet die genannte Engstelle und die Schnittpunkte der Umfänge der kreisrunden Aussparungen 24 bilden die Sicherungsmittel 16.

Der Drehkörper 23 weist zwei Ausnehmungen 31 auf, die in Form eines Langloches entlang einer Linie verlaufen, die konzentrisch zu Drehkörper, innerhalb des Drehkörpers 23 verläuft. Diese Ausnehmungen 31 dienen dazu einen elastischen Bereich zwischen den Ausnehmungen 31 und dem Außenumfang des Drehkörpers 23 zu schafften, der nachgibt, wenn sich die Sicherungsmittel 16 über diesen Bereich bewegen.

Wie ebenfalls gezeigt ist, weist der Drehkörper 23 an der, dem Hinterkopfband 5 gegenüberliegenden Seite, eine Außenverzahnung 25 auf. Außerdem sind Aussparungen 24 auf der dem Hinterkopfband 5 zugewandten Seite vorgesehen, welche eine Innenverzahnung 26 aufweisen, die in Arretierstellung 8 mit der Außenverzahnung 25 in Eingriff steht und zusammen das Arretiermittel 18 bilden.

Aus den Figuren 3 und 5 geht ebenfalls hervor, dass der Drehkörper 23 an der Seite, die dem Hinterkopfband 5 zugewandt ist, eine Drehkörperaussparung 27 aufweist, die einen Teil von Winkelstelleinrichtungen 17 in der Verstellstellung 7 aufnimmt, wobei zumindest eine Seite der Drehkörperaussparung 27 einen Endanschlag 28 für die Drehbewegung des jeweiligen Drehgelenks 6 bildet.

Über solche Winkelstelleinrichtungen 17 sind die Winkelstellungen des Hinterkopfbandes 5 relativ zum Vorderkopfband 4 fest definiert. Im gezeigten Beispielsfall handelt es sich um drei diskrete Winkelstellungen. Hierbei kommen die Winkelstelleinrichtungen 17 vor Arretiermitteln 18 in Eingriff, wenn die Drehgelenke 6 von der Verstellstellung 7 in die Arretierstellung 8 überführt werden.

Wie gezeigt ist, sind die Winkelstelleinrichtungen 17 durch längliche Zapfen gebildet, die vom Vorderkopfband 4 und Hinterkopfband 5 abstehen, wobei die Vorderkopfbandzapfen 19 in der Verstellstellung 7 versetzt zu den Hinterkopfbandzapfen 20 angeordnet sind, und wobei die Vorderkopfbandzapfen 19 und die Hinterkopfbandzapfen 20 in der Arretierstellung 8 ineinandergreifen.

Ebenfalls sind Arretiermittel 18 vorgesehen, die durch eine Verzahnung gebildet sind, wobei die Verzahnung den Winkelstelleinrichtungen 17 bezüglich der Drehachse 10 des Drehgelenks 6 gegenüberliegt.

**Figur 6** zeigt das zuvor beschriebene Hinterkopfband-Gelenkteil 12 am Ende des Hinterkopfbandes (5) von der Seite des Vorderkopfbandes 4 aus.

Die **Figur 8** zeigt eine Atemschutzhaube 29, die durch die zuvor beschriebene Kopfaufhängung 1 auf dem Kopf eines Trägers 32 befestigt ist. Hierzu ist die Kopfaufhängung 1 über Verbindungsmittel 33 mit der Atemschutzhaube 29 verbunden. Die Verbindungsmittel 33 sind im Bereich des Hinterkopfes durch Schlitze gebildet, in denen Gegenverbindungsmittel an der Atemschutzhaube 29 eingreifen und in Längsrichtung beweglich gelagert sind. Entsprechende Verbindungsmittel 33 sind in Figur 7 gezeigt.

Weiter ist gezeigt, dass eine entsprechende Atemschutzhaube 29 vorzugsweise ein Visier 34 aufweist, wobei das Visier 34 im Blickfeld klar und durchsichtig ist. Das Visier 34 ist über zwei zusätzliche Drehgelenke 37, direkt oder indirekt, mit der Kopfaufhängung 1 verbunden und kann über die zusätzlichen Drehgelenke 37 nach oben geklappt werden. Hierbei ist es von Vorteil, wenn der durchsichtige Teil des Visiers 34 leicht gewechselt werden kann, um es bei Verschmutzungen gegen ein neues Teil auszutauschen. Hierzu ist der durchsichtige Teil vorzugsweise aus einem klaren, durchsichtigen Kunststoffteil gefertigt.

Des Weiteren weist die Atemschutzhaube 29 ein Haubentuch 35 auf, dass die Atemschutzhaube 29 zumindest bereichsweise in der Art zur Umgebung hin abgrenzt, dass ein Überdruck in der Haube entsteht, der verhindert, dass Partikel oder Schadstoffe von außen in den Innenbereich der Atemschutzhaube 29 eindringen. Hierzu wird die Atemschutzhaube 29 über einen Druckluftanschluss 36 mit Druckluft versorgt.

## Patentansprüche

1. Kopfaufhängung (1) für eine Kopfbedeckung (2), vorzugsweise für eine Atemschutzhaube (29), umfassend ein Kopfband (3) mit einem Vorderkopfband (4), welches die Vorderseite des Kopfes eines Trägers umläuft, und einem Hinterkopfband (5), welches sich auf der Hinterseite des Kopfes des Trägers befindet, wobei das Hinterkopfband (5) über zwei Drehgelenke (6) mit dem Vorderkopfband (4) verbunden ist, **dadurch gekennzeichnet, dass** die Drehgelenke (6) in einer jeweiligen Verstellstellung (7) zumindest bereichsweise frei drehbar sind und in einer jeweiligen Arretierstellung (8) nicht drehbar sind, wobei die Drehgelenke (6) jeweils durch eine Relativbewegung (30) der Drehachse (10) des jeweiligen Drehgelenks (6) von der Verstellstellung (7) in die Arretierstellung (8) und zurück überführbar sind.

2. Kopfaufhängung (1) für eine Kopfbedeckung (2) nach Anspruch 1, **dadurch gekennzeichnet, dass** die Drehgelenke (6) jeweils durch eine geradlinige Relativbewegung (30) der Drehachse (10) des jeweiligen Drehgelenks (6), vorzugsweise über ein Gleiten in einem Langloch (9) und/oder einer Längsnut, von der Verstellstellung (7) in die Arretierstellung (8) und zurück überführbar sind.

3. Kopfaufhängung (1) für eine Kopfbedeckung (2) nach Anspruch 1 oder 2, **dadurch gekennzeichnet, dass** die Drehgelenke (6) jeweils durch die Relativbewegung (30) eines Teils des jeweiligen Drehgelenks (6) am Hinterkopfband (5) in Richtung der Blickrichtung des Trägers (14) von der Arretierstellung (8) in die Verstellstellung (7) überführbar sind.

4. Kopfaufhängung (1) für eine Kopfbedeckung (2) nach einem der vorigen Ansprüche, **dadurch gekennzeichnet, dass** das Hinterkopfband (5) ein Längenverstellmittel (15) aufweist, mittels dessen die Länge und damit der Innenumfang des Kopfbandes (3) verstellt werden kann.

5. Kopfaufhängung (1) für eine Kopfbedeckung (2) nach einem der vorigen Ansprüche, **dadurch gekennzeichnet, dass** die Drehgelenke (6) jeweils ein Sicherungsmittel (16), insbesondere in Form einer Engstelle in einer Längsnut oder einem Langloch, aufweisen, das der Überführung des jeweiligen Drehgelenks (6) von der Verstellstellung (7) in die Arretierstellung (8) mit einer Sicherungskraft entgegenwirkt, die aufgebracht werden muss, um das Sicherungsmittel (16) zu überwinden und das Drehgelenk (6) von der Arretierstellung (8) in die Verstellstellung (7) zu überführen.

6. Kopfaufhängung (1) für eine Kopfbedeckung (2) nach einem der vorigen Ansprüche, **dadurch gekennzeichnet, dass** die Drehgelenke (6) jeweils eine Winkelstelleinrichtung (17) aufweisen, über welche mehrere Winkelstellungen des Hinterkopfbandes (5) relativ zum Vorderkopfband (4), vorzugsweise drei oder zwei Winkelstellungen, fest definiert sind, vorzugsweise wobei die Winkelstelleinrichtungen (17) vor Arretiermitteln (18) in Eingriff kommen, wenn die Drehgelenke (6) von der Verstellstellung (7) in die Arretierstellung (8) überführt werden.

7. Kopfaufhängung (1) für eine Kopfbedeckung (2) nach einem der vorigen Ansprüche, **dadurch gekennzeichnet, dass** die Winkelstelleinrichtungen (17) durch längliche Zapfen gebildet sind, die vom Vorderkopfband (4) und Hinterkopfband (5) abstehen, vorzugsweise wobei die Vorderkopfbandzapfen (19) in der Verstellstellung (7) versetzt zu den Hinterkopfbandzapfen (20) angeordnet sind, wobei Vorderkopfbandzapfen (19) und Hinterkopfbandzapfen (20) in der Arretierstellung (8) ineinandergreifen.

8. Kopfaufhängung (1) für eine Kopfbedeckung (2) nach einem der vorigen Ansprüche, **dadurch gekennzeichnet, dass** Arretiermittel (18) vorgesehen sind, die durch eine Verzahnung gebildet sind, vorzugsweise wobei die Verzahnung den Winkelstelleinrichtungen (17) bezüglich der Drehachse (10) des Drehgelenks (6) gegenüberliegt.

9. Kopfaufhängung (1) für eine Kopfbedeckung (2) nach einem der vorigen Ansprüche, **dadurch gekennzeichnet, dass** die Drehgelenke (6) im Wesentlichen mittig zwischen Oberkante (21) und Unterkante (22) des Vorderkopfbands (4) angeordnet sind.

10. Kopfaufhängung (1) für eine Kopfbedeckung (2) nach einem der vorigen Ansprüche, **dadurch gekennzeichnet, dass** die Drehgelenke (6) jeweils einen Drehkörper (23), der vorzugsweise gegenüber dem Vorderkopfband (4) vorsteht, und zwei sich überschneidende Aussparungen (24), vorzugsweise am Hinterkopfband (5), aufweisen.

11. Kopfaufhängung (1) für eine Kopfbedeckung (2) nach einem der vorigen Ansprüche, **dadurch gekennzeichnet, dass** die Drehgelenke (6) jeweils einen Drehkörper (23) aufweisen, der an der dem Hinterkopfband (5) gegenüberliegenden Seite, eine Außenverzahnung (25) aufweist, und dass Aussparungen (24) auf der dem Hinterkopfband (5) zugewandten Seite eine Innenverzahnung (26) aufweisen, die in Arretierstellung (8) mit der Außenverzahnung (25) in Eingriff steht und zusammen Arretiermittel (18) bilden.

12. Kopfaufhängung (1) für eine Kopfbedeckung (2) nach einem der vorigen Ansprüche, **dadurch gekennzeichnet, dass** die Drehgelenke (6) jeweils einen Drehkörper (23) aufweisen, der an der Seite, die dem Hinterkopfband (5) zugewandt ist, eine Drehkörperaussparung (27) aufweist, die einen Teil von Winkelstelleinrichtungen (17) in der Verstellstellung (7) aufnimmt, vorzugsweise wobei zumindest eine Seite der Drehkörperaussparung (27) einen Endanschlag (28) für die Drehbewegung des jeweiligen Drehgelenks (6) bildet.

13. Atemschutzhaube (29) mit einer Kopfaufhängung (1) nach einem der vorigen Ansprüche.

14. Verfahren zum Befestigen einer Kopfbedeckung (2), insbesondere einer Atemschutzhaube (29), mit einer Kopfaufhängung (1) nach einem der Ansprüche 1 bis 12, wobei der Träger die Winkelstellung des Hinterkopfbandes (5) relativ zum Vorderkopfband (4) entsprechend seiner Kopfform und/oder Kopfgröße einstellt und die Drehgelenke (6) anschließend über die Relativbewegung (30), insbesondere eine gradlinige Relativbewegung (30), der Drehgelenke (6) von der Verstellstellung (7) in die Arretierstellung (8) überführt.

15. Verfahren nach Anspruch 14, **dadurch gekennzeichnet, dass** der Träger die Drehgelenke (6) von der Verstellstellung (7) in die Arretierstellung (8) überführt, indem er die Länge und damit den Innenumfang des Kopfbandes (3) über das Längeneinstellmittel (15) reduziert.

## Claims

1. Head suspension (1) for a headgear (2), preferably for a respiratory protection hood (29), comprising a headband (3) with a front headband (4) which runs around the front of the head of a wearer, and a rear headband (5) which is located on the rear of the head of the wearer, wherein the rear headband (5) is connected to the front headband (4) by means of two swivel joints (6), **characterized in that** the swivel joints (6) in a respective adjustment position (7) at least in certain areas are freely rotatable and in a respective locking position (8) are not rotatable, wherein the swivel joints (6), each with a relative movement (30) of the rotating axis (10) of the respective swivel joint (6), are transferable from the adjustment position (7) into the locking position (8) and back.

2. Head suspension (1) for a headgear (2) according to claim 1, **characterized in that** the swivel joints (6) can each be transferred from the adjustment position (7) into the locking position (8) and back by a rectilinear relative movement (30) of the rotating axis (10) of the respective swivel joint (6), preferably by sliding in an elongated hole (9) and/or a longitudinal groove.

3. Head suspension (1) for a headgear (2) according to claim 1 or 2, **characterized in that** the swivel joints (6) can each be transferred from the locking position (8) into the adjustment position (7) by the relative movement (30) of a part of the respective swivel joint (6) on the rear headband (5) in the viewing direction of the wearer (14).

4. Head suspension (1) for a headgear (2) according to one of the preceding claims, **characterized in that** the rear headband (5) has a length adjustment means (15), by means of which the length and thus the inner circumference of the headband (3) can be adjusted.

5. Head suspension (1) for a headgear (2) according to one of the preceding claims, **characterized in that** the swivel joints (6) each have a securing means (16), in particular in the form of a constriction in a longitudinal groove or an elongate hole, which counteracts the transfer of the respective swivel joint (6) from the adjustment position (7) into the locking position (8) with a securing force, which must applied in order to overcome the securing means (16) and to transfer the swivel joint (6) from the locking position (8) into the adjustment position (7).

6. Head suspension (1) for a headgear (2) according to one of the preceding claims, **characterized in that** the swivel joints (6) each have an angle adjusting device (17), by means of which a plurality of angular positions of the rear headband (5) relative to the front headband (4), preferably three or two angular positions, are fixedly defined, preferably wherein the angle adjusting devices (17) coming into engagement before locking means (18) when the swivel joints (6) are transferred from the adjustment position (7) into the locking position (8).

7. Head suspension (1) for a headgear (2) according to one of the preceding claims, **characterized in that** the angle adjusting devices (17) are formed by elongate pins which project from the front headband (4) and the rear headband (5), preferably wherein the front headband pins (19) in the adjustment position (7) are arranged offset relative to the rear headband pins (20), wherein front headband pins (19) and rear headband pins (20) engage in the locking position (8).

8. Head suspension (1) for a headgear (2) according to one of the preceding claims, **characterized in that** locking means (18) are provided which are formed by a toothing, preferably wherein the toothing is situated opposite the angle adjusting devices (17) with respect to the rotating axis (10) of the swivel joint (6).

9. Head suspension (1) for a headgear (2) according to one of the preceding claims, **characterized in that** the swivel joints (6) essentially arranged centrally between the upper edge (21) and the lower edge (22) of the front headband (4).

10. Head suspension (1) for a headgear (2) according to one of the preceding claims, **characterized in that** the swivel joints (6) each have a swivel body (23) which preferably projects relative to the front headband (4), and two intersecting recesses (24), preferably on the rear headband (5).

11. Head suspension (1) for a headgear (2) according to one of the preceding claims, **characterized in that** the swivel joints (6) each have a swivel body (23) which has an external toothing (25) on the side opposite the rear headband (5), and that recesses (24) on the side facing the rear headband (5), have an internal toothing (26) that engages with the external toothing (25) in the locking position (8) and together form the locking means (18).

12. Head suspension (1) for a headgear (2) according to one of the preceding claims, **characterized in that** the swivel joints (6) each have a swivel body (23) which, on the side facing the rear headband (5), has a swivel body recess (27) which receives a part of the angle adjusting devices (17) in the adjustment position (7), preferably wherein at least one side of the swivel body recess (27) forming an end stop (28) for the rotational movement of the respective swivel joint (6).

13. Respiratory protection hood (29) with a head suspension (1) according to one of the preceding claims.

14. Method for fastening a headgear (2), in particular a respiratory protection hood (29), with a head suspension (1) according to one of claims 1 to 12, wherein the wearer adjusts the angular position of the rear headband (5) relative to the front headband (4) according to their head shape and/or head size, and subsequently transfers the swivel joints (6) by the relative movement (30), in particular a rectilinear relative movement (30), of the swivel joints (6) from the adjustment position (7) into the locking position (8) .

15. Method according to claim 14, **characterized in that** the wearer transfers the swivel joints (6) from the adjustment position (7) into the locking position (8) by reducing the length and thus the inner circumference of the headband (3) by means of the length adjusting means (15).

## Revendications

1. Suspension de tête (1) pour un couvre-chef (2), de préférence pour une cagoule de protection respiratoire (29), comprenant un bandeau pour tête (3) comportant un bandeau pour tête avant (4), lequel entoure le côté avant de la tête d'une personne porteuse, et un bandeau pour tête arrière (5), lequel se situe sur le côté arrière de la tête de la personne porteuse, dans laquelle le bandeau pour tête arrière (5) est relié au bandeau pour tête avant (4) par l'intermédiaire de deux articulations tournantes (6), **caractérisée en ce que** les articulations tournantes (6) peuvent tourner librement au moins dans certaines régions dans une position de réglage (7) respective et ne peuvent pas tourner dans une position de verrouillage (8) respective, dans laquelle les articulations tournantes (6) peuvent être transférées de la position de réglage (7) à la position de verrouillage (8) et inversement respectivement par un mouvement relatif (30) de l'axe de rotation (10) de l'articulation tournante (6) respective.

2. Suspension de tête (1) pour un couvre-chef (2) selon la revendication 1, **caractérisée en ce que** les articulations tournantes (6) peuvent être transférées de la position de réglage (7) à la position de verrouillage (8) et inversement respectivement par un mouvement relatif (30) rectiligne de l'axe de rotation (10) de l'articulation tournante (6) respective, de préférence par l'intermédiaire d'un coulissement dans un trou oblong (9) et/ou une rainure longitudinale.

3. Suspension de tête (1) pour un couvre-chef (2) selon la revendication 1 ou 2, **caractérisée en ce que** les articulations tournantes (6) peuvent être transférées de la position de verrouillage (8) à la position de réglage (7) respectivement par le mouvement relatif (30) d'une partie de l'articulation tournante (6) respective sur le bandeau pour tête arrière (5) en direction de la direction du regard de la personne porteuse (14).

4. Suspension de tête (1) pour un couvre-chef (2) selon l'une des revendications précédentes, **caractérisée en ce que** le bandeau pour tête arrière (5) présente un moyen de réglage de longueur (15) au moyen duquel la longueur et donc la circonférence intérieure du bandeau pour tête (3) peuvent être réglées.

5. Suspension de tête (1) pour un couvre-chef (2) selon l'une des revendications précédentes, **caractérisée en ce que** les articulations tournantes (6) présentent respectivement un moyen de blocage (16), en particulier sous la forme d'un rétrécissement dans une rainure longitudinale ou un trou oblong, qui agit à l'encontre du transfert de l'articulation tournante (6) respective de la position de réglage (7) à la position de verrouillage (8) avec une force de blocage qui doit être appliquée pour surmonter le moyen de blocage (16) et pour transférer l'articulation tournante (6) de la position de verrouillage (8) à la position de réglage (7).

6. Suspension de tête (1) pour un couvre-chef (2) selon l'une des revendications précédentes, **caractérisée en ce que** les articulations tournantes (6) présentent respectivement un dispositif de réglage angulaire (17) par l'intermédiaire duquel plusieurs positions angulaires du bandeau pour tête arrière (5) par rapport au bandeau pour tête avant (4) sont définies de manière fixe, de préférence trois ou deux positions angulaires, de préférence dans laquelle les dispositifs de réglage angulaire (17) viennent en prise devant des moyens de verrouillage (18) lorsque les articulations tournantes (6) sont transférées de la position de réglage (7) à la position de verrouillage (8).

7. Suspension de tête (1) pour un couvre-chef (2) selon l'une des revendications précédentes, **caractérisée en ce que** les dispositifs de réglage angulaire (17) sont formés par des tourillons allongés qui font saillie depuis le bandeau pour tête avant (4) et le bandeau pour tête arrière (5), de préférence dans laquelle, dans la position de réglage (7), les tourillons de bandeau pour tête avant (19) sont disposés de manière à être décalés par rapport aux tourillons de bandeau pour tête arrière (20), dans laquelle les tourillons de bandeau pour tête avant (19) et les tourillons de bandeau pour tête arrière (20) viennent en prise les uns dans les autres dans la position de verrouillage (8).

8. Suspension de tête (1) pour un couvre-chef (2) selon l'une des revendications précédentes, **caractérisée en ce que** des moyens de verrouillage (18) formés par une denture sont prévus, de préférence dans laquelle la denture est opposée aux dispositifs de réglage angulaire (17) par rapport à l'axe de rotation (10) de l'articulation tournante (6).

9. Suspension de tête (1) pour un couvre-chef (2) selon l'une des revendications précédentes, **caractérisée en ce que** les articulations tournantes (6) sont disposées sensiblement au centre entre le bord supérieur (21) et le bord inférieur (22) du bandeau pour tête avant (4).

10. Suspension de tête (1) pour un couvre-chef (2) selon l'une des revendications précédentes, **caractérisée en ce que** les articulations tournantes (6) présentent respectivement un corps tournant (23), lequel fait saillie de préférence par rapport au bandeau pour tête avant (4), et deux évidements (24) se chevauchant, de préférence sur le bandeau pour tête arrière (5).

11. Suspension de tête (1) pour un couvre-chef (2) selon l'une des revendications précédentes, **caractérisée en ce que** les articulations tournantes (6) présentent respectivement un corps tournant (23), lequel présente une denture extérieure (25) sur le côté opposé au bandeau pour tête arrière (5), **et en ce que** les évidements (24) présentent, sur le côté tourné vers le bandeau pour tête arrière (5), une denture intérieure (26) qui est en prise avec la denture extérieure (25) dans la position de verrouillage (8) et forme avec celle-ci des moyens de verrouillage (18).

12. Suspension de tête (1) pour un couvre-chef (2) selon l'une des revendications précédentes, **caractérisée en ce que** les articulations tournantes (6) présentent respectivement un corps tournant (23) qui présente un évidement de corps tournant (27) sur le côté tourné vers le bandeau pour tête arrière (5), lequel évidement reçoit une partie des dispositifs de réglage angulaire (17) dans la position de réglage (7), de préférence dans laquelle au moins un côté de l'évidement de corps tournant (27) forme une butée d'extrémité (28) pour le mouvement de rotation de l'articulation tournante (6) respective.

13. Cagoule de protection respiratoire (29) comportant une suspension de tête (1) selon l'une des revendications précédentes.

14. Procédé permettant de fixer un couvre-chef (2), en particulier une cagoule de protection respiratoire (29), avec une suspension de tête (1) selon l'une des revendications 1 à 12, dans lequel la personne porteuse règle la position angulaire du bandeau pour tête arrière (5) par rapport au bandeau pour tête avant (4) conformément à sa forme de tête et/ou sa taille de tête et transfère ensuite les articulations tournantes (6) de la position de réglage (7) à la position de verrouillage (8) par l'intermédiaire du mouvement relatif (30), en particulier d'un mouvement relatif (30) rectiligne, des articulations tournantes (6).

15. Procédé selon la revendication 14, **caractérisé en ce que** la personne porteuse transfère les articulations tournantes (6) de la position de réglage (7) à la position de verrouillage (8) en réduisant la longueur et donc la circonférence intérieure du bandeau pour tête (3) par l'intermédiaire du moyen de réglage de longueur (15).
